# EUROPEAN PATENT APPLICATION

(11) **EP 2 177 219 A1**
(43) Date of publication of application: **21.04.2010**
(21) Application number: 08792077.3
(22) Date of filing: 01.08.2008
(51) Int. Cl.: A61K 31/426, A61K 33/42, A61P 19/08

(54) **PREVENTIVE AGENT OR THERAPEUTIC AGENT FOR DISEASE CAUSED BY ABNORMAL BONE METABOLISM**

(30) Priority: 02.08.2007 US 935262 P
(71) Applicant: Teijin Pharma Limited, Tokyo 100-0013 (JP)
(72) Inventor: OCHIAI, Eiji, Hino-shi Tokyo 191-0065 (JP); NISHIGA, Miyuki, Hino-shi Tokyo 191-0065 (JP); TAKAGI, Kenichiro, Hino-shi Tokyo 191-0065 (JP); AZUMA, Yoshiaki, Hino-shi Tokyo 191-0065 (JP)
(74) Representative: Hallybone, Huw George
(86) International application number: PCT/JP2008/063864
(87) International publication number: WO 2009/017222

(57) **Abstract**

An object of the present invention is to provide a preventive drug and a therapeutic drug for diseases caused by abnormal bone metabolism, especially osteoporosis, which is more effective than conventional drugs. Combined use of N-hydroxy-4-{5-[4-(5-isopropyl-2-methyl-1,3-thiazol-4-yl)phenoxy]pentoxy}-benzamidine, or a salt thereof; and at least one compound selected from the group consisting of etidronic acid, clodronic acid, pamidronic acid, tiludronic acid, risedronic acid, minodronic acid, ibandronic acid, zoledronic acid, and salts thereof can exert higher bone resorption inhibitory effect and provide a preventive effect and a therapeutic effect for diseases caused by abnormal bone metabolism, especially osteoporosis as compared with administration of the respective agents respectively.

## Description

### Technical Field

The present invention relates to a preventive or therapeutic agent for diseases caused by abnormal bone metabolism, particularly to a preventive or therapeutic agent for osteoporosis, and specifically to a preventive or therapeutic agent for diseases caused by abnormal bone metabolism, especially osteoporosis, containing, as active ingredients, N-hydroxy-4-{5-[4-(5-isopropyl-2-methyl-1,3-thiazol-4-yl)phenoxy]pentoxy}-benzamidine, or a salt thereof, and at least one compound selected from the group consisting of etidronic acid, clodronic acid, pamidronic acid, tiludronic acid, risedronic acid, minodronic acid, ibandronic acid, zoledronic acid, and salts thereof

### Background Art

N-hydroxy-4-{5-[4-(5-isopropyl-2-methyl-1,3-thiazol-4-yl)phenoxy]pentoxy}-benzamidine represented by formula (I) or a salt thereof has a bone resorption inhibitory effect and a bone formation promoting effect, and is expected as a therapeutic or preventive agent for osteoporosis.

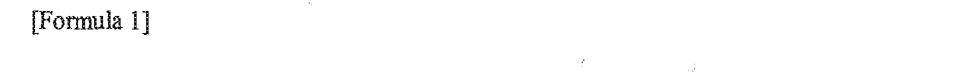

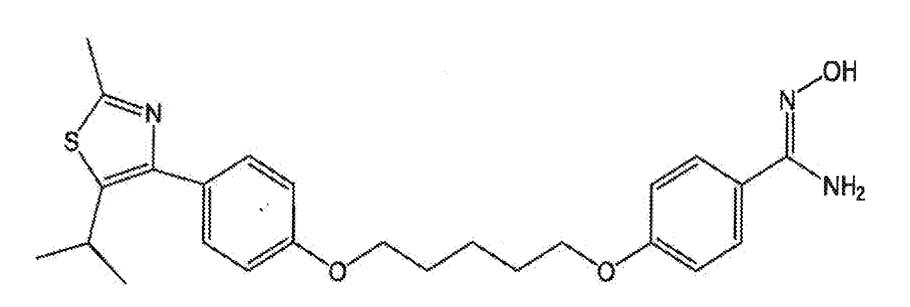

N-hydroxy-4-{5-[4-(5-isopropyl-2-methyl-1,3-thiazol-4-yl)phenoxy]pentoxy}-benzamidine, or a salt thereof is disclosed in Patent Document 1 and Non-patent Document 1.

Etidronic acid, clodronic acid, pamidronic acid, tiludronic acid, risedronic acid, minodronic acid, ibandronic acid, zoledronic acid, or salts thereof are considered to act specifically on activated osteoclasts and suppress bone resorption by suppressing their activity, and used as therapeutics agent for diseases caused by abnormal calcium metabolism such as osteoporosis and the like.

A large number of therapeutic agents for osteoporosis are currently available. Therapeutic effects of combined use of these agents are varied, however. For example, a combination of PTH (parathyroid hormone) and a certain bisphosphonic acid is known to cancel their therapeutic effect each other. Although both agents are representative therapeutic agents for osteoporosis, it has been reported as the results of a preclinical study and a clinical study that their effects were reduced when they were administered in combination as compared with the effects obtained when they were administered alone, respectively (Non-patent Documents 2 and 3). It is not easily conceivable to discover a therapeutic or preventive method having higher efficacy using an existing or novel therapeutic agent for osteoporosis, as is also considered from this example.
Patent Document 1: Japanese Unexamined Patent Application Publication No. 2004-537549
Non-patent Document 1:Lee Sung-eun, Synthesis and Biological Activity of Natural Products and Designed New Hybrid Compounds for the Treatment of LTB4 Related Disease, Graduate School of Pusan National University, Department of Science, Doctoral dissertation, 1999. 8
Non-patent Document 2: R Samadfam et al., Endocrinology, Vol. 148, No. 6, 2007
Non-patent Document 3: DM Black et al., The New England Journal of Medicine, Vol. 349, No. 13, 2003

### Disclosure of the Invention

### Problems to be Solved by the Invention

An object of the present invention is to provide a drug or a therapeutic or preventive method that is more effective as a therapeutic or preventive agent and a therapeutic or preventive method for diseases caused by abnormal bone metabolism, especially osteoporosis than existing drugs.

The present inventors have keenly studied ingredients that are effective as a therapeutic or preventive agent for diseases caused by abnormal bone metabolism, especially osteoporosis, and found that combined use of specific drugs, each of which can be used alone, can provide a therapeutic or preventive agent or a therapeutic or preventive method for diseases caused by abnormal bone metabolism, especially osteoporosis that has extremely high efficacy.

In other words, the present invention is a preventive or therapeutic agent or a preventive or therapeutic method for diseases caused by abnormal bone metabolism, especially osteoporosis, containing as active ingredients, the following (a) and (b):
(a) N-hydroxy-4-{5-[4-(5-isopropyl-2-methyl-1,3-thiazol-4-yl)phenoxy]pentoxy}-benzamidine, or a salt thereof; and
(b) at least one compound selected from the group consisting of etidronic acid, clodronic acid, pamidronic acid, tiludronic acid, risedronic acid, minodronic acid, ibandronic acid, zoledronic acid, and salts thereof.

Further, the present invention is a preventive or therapeutic agent for diseases caused by abnormal bone metabolism, especially osteoporosis, containing as active ingredients (a) and (b), in a form of a combination drug or independent single drugs.

### Effect of the Invention

The present invention is a preventive or therapeutic drug or a preventive or therapeutic method for diseases caused by abnormal bone metabolism, especially osteoporosis, wherein N-hydroxy-4-{5-[4-(5-isopropyl-2-methyl-1,3-thiazol-4-yl)phenoxy]pentoxy}-benzamidine, or a salt thereof; and at least one compound selected from the group consisting of etidronic acid, clodronic acid, pamidronic acid, tiludronic acid, risedronic acid, minodronic acid, ibandronic acid, zoledronic acid, and salts thereof; are administered in combination, by which an extremely strong preventive or therapeutic effect can be obtained as compared with the effect obtained by either ingredient alone.

### Brief Description of Drawings

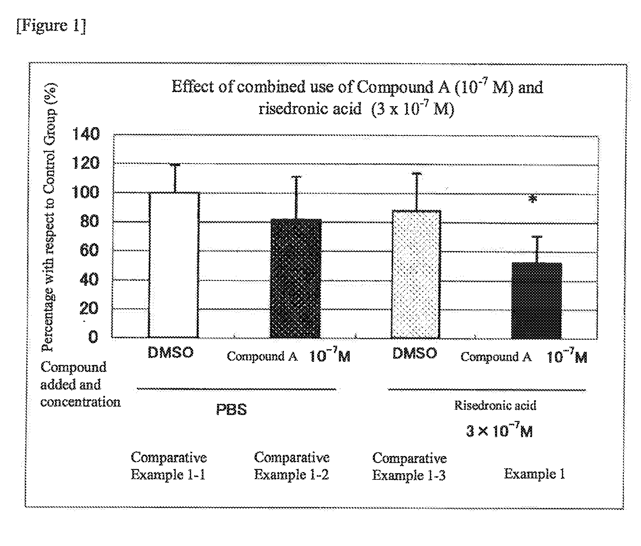

Fig. 1 is a graph showing an inhibitory effect on the number of formed osteoclast-like cells of a solvent, N-hydroxy-4-{5-[4-(5-isopropyl-2-methyl-1,3-thiazol-4-yl)phenoxy]pentoxy}-benzamidine (Compound A) and risedronic acid that were used respectively or in combination of two of them in the experimental system in which osteoclast-like cells were formed by adding 1α,2S(OH)₂D₃to mouse bone marrow-derived cells and culturing the cells for 7 days.

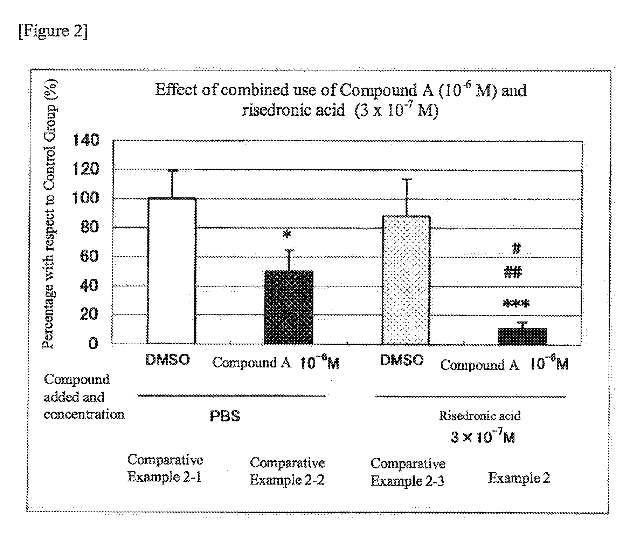

Figure 2 is a graph showing the inhibitory effect on the number of formed osteoclast-like cells of a solvent, N-hydroxy-4-{5-[4-(5-isopropyl-2-methyl-1,3-thiazol-4-yl)phenoxy]pentoxy}-benzamidine (Compound A) and risedronic acid that were used respectively or in combination of two of them in the experimental system in which osteoclast-like cells were formed by adding 1α,25(OH)₂D₃ to mouse bone marrow-derived cells and culturing the cells for 7 days.

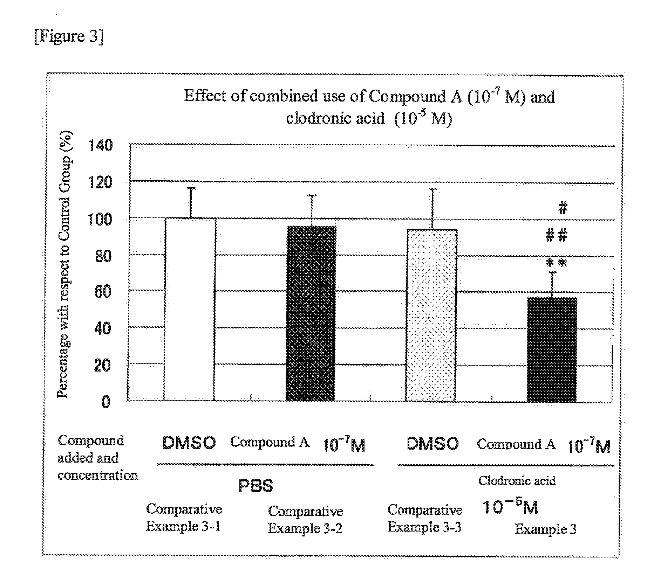

Figure 3 is a graph showing the inhibitory effect on the number of formed osteoclast-like cells of a solvent, N-hydroxy-4-{5-[4-(5-isopropyl-2-methyl-1,3-thiazol-4-yl)phenoxy]pentoxy}-benzamidine (Compound A) and risedronic acid that were used respectively or in combination of two of them in the experimental system in which osteoclast-like cells were formed by adding 1α,25(OH)₂D₃ to mouse bone marrow-derived cells and culturing the cells for 7 days.

### Best Mode for Carrying out the Invention

N-hydroxy-4-{5-[4-(5-isopropyl-2-methyl-1,3-thiazol-4-yl)phenoxy]pentoxy}-benzamidine, or a salt thereof used in the present invention has an excellent bone resorption inhibitory effect and a bone formation promoting effect and is expected as a therapeutic or preventive agent for osteoporosis.

N-hydroxy-4-{5-[4-(5-isopropyl-2-methyl-1,3-thiazol-4-yl)phenoxy]pentoxy}-benzamidine may be manufactured by known methods such as the method described in Non-patent Document 1 and the like.

Examples of the salt of N-hydroxy-4-{5-[4-(5-isopropyl-2-methyl-1,3-thiazol-4-yl)phenoxy]pentoxy}-benzamidine include pharmaceutically acceptable salts obtained by using inorganic acids (hydrochloric acid, hydrobromic acid, sulfuric acid and phosphoric acid) and organic acids (citric acid, acetic acid, lactic acid, tartaric acid, fumaric acid, formic acid, propionic acid, oxalic acid, trifluoroacetic acid, methanesulfonic acid, ethanesulfonic acid, benzoic acid, maleic acid, gluconic acid, glycolic acid, succinic acid, 4-toluenesulfonic acid, galacturonic acid, embonic acid, glutamic acid or aspartic acid). In the present invention, hydrochloric acid is preferably used as an inorganic acid and methanesulfonic acid or ethanesulfonic acid as an organic acid.

The dose of N-hydroxy-4-{5-[4-(5-isopropyl-2-methyl-1,3-thiazol-4-yl)phenoxy]pentoxy}-benzamidine, or a salt thereof is an effective dose for the prevention or treatment of osteoporosis and depends on the age and body weight of a patient, the type of combination therapy, the frequency of treatment, the type of desired effect, the administration method or the like, and thus is not determined unconditionally. Generally, when it is used as a therapeutic or preventive agent, the ordinary dose administered may be used.

4-{5-[4-(5-Isopropyl-2-methyl-1,3-thiazol-4-yl)phenoxy]pentoxy}-benzamidine, or a salt thereof, which is a dehydroxy form of N-hydroxy-4-{5-[4-(5-isopropyl-2-methyl-1,3-thiazol-4-yl)phenoxy]pentoxy}-benzamidine, also has excellent bone resorption inhibitory effect and bone formation promoting effect and is expected as a preventive or therapeutic agent for osteoporosis. Likewise, 4-{5-[4-(5-isopropyl-2-methyl-1,3-thiazol-4-yl)phenoxy]pentoxy}-benzamidine, or a salt thereof is also used in combination with at least one compound selected from the group consisting or etidronic acid, clodronic acid, pamidronic acid, tiludronic acid, risedronic acid, minodronic acid, ibandronic acid, zoledronic acid, and salts thereof. The combined use provides a stronger effect than the use of the respective ingredients alone as a preventive or therapeutic agent or a preventive or therapeutic method for osteoporosis.

Among etidronic acid, clodronic acid, pamidronic acid, tiludronic acid, risedronic acid, minodronic acid, ibandronic acid, zoledronic acid, or a salt thereof used in the present invention, clodronic acid or risedronic acid is preferable, and risedronic acid is more preferable. When these are used in combination with N-hydroxy-4-{5-[4-(5-isopropyl-2-methyl-1,3-thiazol-4-yl)phenoxy]pentoxy}-benzamidine, or a salt thereof in the present invention, one kind or a mixture of two or more kinds in an arbitrary ratio may be used. These are manufactured by known methods.

Examples of the salt of etidronic acid, clodronic acid, pamidronic acid, tiludronic acid, risedronic acid, minodronic acid, ibandronic acid or zoledronic acid used in the present invention include pharmaceutically acceptable salts obtained by using inorganic acids (hydrochloric acid, hydrobromic acid, sulfuric acid and phosphoric acid) and organic acids (citric acid, acetic acid, lactic acid, tartaric acid, fumaric acid, formic acid, propionic acid, oxalic acid, trifluoroacetic acid, methanesulfonic acid, benzoic acid, maleic acid, gluconic acid, glycolic acid, succinic acid, 4-toluenesulfonic acid, galacturonic acid, embonic acid, glutamic acid or aspartic acid) or inorganic bases (alkaline metal salts of sodium, potassium and the like, alkaline-earth metal salts of magnesium, calcium and the like, and metal salts of aluminum, zinc and the like) or organic bases (such as ammonia, triethylamine, diethylamine, ethylenediamine, propanediamine, pyrrolidine, piperidine, piperazine, pyridine, lysine, choline, ethanolamine, diethanolamine, N,N-dimethylethanolamine, 4-hydroxypiperidine, glucosamine, N-methylglucamine and the like).

The preventive or therapeutic agent for osteoporosis according to the present invention provides a drug having an effect to improve bone mineral density and bone strength equal to or higher than that of conventionally known therapeutic agents for osteoporosis by using two types of ingredients having different mode of actions, namely, at least one compound selected from the group consisting of etidronic acid, clodronic acid, pamidronic acid, tiludronic acid, risedronic acid, minodronic acid, ibandronic acid, zoledronic acid, and salts thereof; and N-hydroxy-4-{5-[4-(5-isopropyl-2-methyl-1,3-thiazol-4-yl)phenoxy]pentoxy}-benzamidine, or a salt thereof, as active ingredients. The drug according to the present invention is less toxic and has excellent stability. The combined use of the active ingredients allows a reduction in dose as compared with the respective active ingredients used respectively.

The dose of at least one compound selected from the group consisting of etidronic acid, clodronic acid, pamidronic acid, tiludronic acid, risedronic acid, minodronic acid, ibandronic acid, zoledronic acid, and salts thereof is an effective dose for the prevention or treatment of osteoporosis and depends on the age and body weight of a patient, the type of combination therapy, the frequency of treatment, the type of desired effect, the administration method or the like, and thus is not determined unconditionally. Generally, when it is used as a therapeutic or preventive agent, the ordinary dose administered may be used.

The preventive or therapeutic agent or preventive or therapeutic method for osteoporosis according to the present invention may be any, as long as it contains N-hydroxy-4-{5-[4-(5-isopropyl-2-methyl-1,3-thiazol-4-yl)phenoxy]pentoxy}-benzamidine, or a salt thereof; and at least one compound selected from the group consisting of etidronic acid, clodronic acid, pamidronic acid, tiludronic acid, risedronic acid, minodronic acid, ibandronic acid, zoledronic acid, and salts thereof; as active ingredients. These ingredients may be mixed and simultaneously administered, of may be administered separately at the same time or successively or with a certain time interval. When not administered simultaneously, the active ingredients may be administered alternatively, or one agent may be administered continuously and then another agent may be administered, for example.

The drug according to the present invention may be in any form of a drug, as long as it contains N-hydroxy-4-{5-[4-(5-isopropyl-2-methyl-1,3-thiazol-4-yl)phenoxy]pentoxy}-benzamidine, or a salt thereof; and at least one compound selected from the group consisting of etidronic acid, clodronic acid, pamidronic acid, tiludronic acid, risedronic acid, minodronic acid, ibandronic acid, zoledronic acid, and salts thereof; as active ingredients. For example, a combination drug containing both active ingredients may be constituted, or a single drug containing each of the active ingredients may be constituted. Used herein, "combination drug" refers to a preparation containing two or more active ingredients in combination in one preparation, and "single drug" refers to a preparation containing one active ingredient in one preparation. According to the aspect of a single drug, when a plurality of compounds selected from etidronic acid, clodronic acid, pamidronic acid, tiludronic acid, risedronic acid, minodronic acid, ibandronic acid and zoledronic acid or salts thereof are used, the respective compounds may be used as a single drug or a combination drug, but it is preferable to use them as single drugs.

A therapeutic or preventive agent or a therapeutic or preventive method according to the present invention in which both active ingredients are used as single drugs means a drug or a method in which single drugs that can be used respectively are used in combination. Accordingly, drugs containing each of the active ingredients may be in different dosage forms. For example, the forms of the respective drugs may be solid or liquid for both, or a combination of solid and liquid, and are not particularly restricted. When the respective active ingredients are single drugs, they may be in a form of a kit containing a set of both single drugs. Examples of the representative form of the kit include a blister package in which both drugs in the quantities corresponding to a specific period (for example, one week or a longer period) in accordance with an administration schedule are packaged in one sheet. Further, the drugs may be packed in the same package like PTP at the end of manufacturing stage of the drugs or may be placed in the same bag at the time of prescription at a hospital or a pharmacy, and the form of the kit is not particularly limited.

As the combination drug, for example, both active ingredients in the amounts with which the respective ingredients can exhibit their respective effects may be combined to manufacture dosage forms such as tablets, capsules, liquids and solution, and the like. The time of combining the active ingredients to produce a combination drug may be in the stage of manufacturing a dosage form as a combination drug or immediately before administration. When the active ingredients are combined in the stage of manufacturing, the active ingredients in appropriate amounts may be mixed, shaped, and packaged. The method of shaping is not particularly restricted, and the respective agents may be mixed or layered. When a combination drug is prepared immediately before administration, various methods are available: the respective active ingredients are stored separately until immediately before administration, and agents in the liquid form are mixed, or an agent in the solid form such as a tablet, a pill, granules, powder, or a capsule is dissolved in an agent in the liquid form, or agents in the solid form such as granules or powder are mixed together at the time of administration. The method for mixing the ingredients immediately before administration may be conducted manually or use a package that allows mixing the ingredients easily by cutting, drawing, splitting, pulling out or the like. The forms of the combination drug include dosage forms such as tablets, pills, granules, powders, solutions, suspensions, syrups, capsules or the like.

Further, the preventive or therapeutic agent for osteoporosis according to the present invention may be administered daily or intermittently, and once daily or 2 to 3 times daily with a divided dose. When both active ingredients are single drugs, the number of doses for the respective active ingredients may be the same or different from each other. Examples of the administration method includes a method in which N-hydroxy-4-{5-[4-(5-isopropyl-2-methyl-1,3-thiazol-4-yl)phenoxy]pentoxy}-benzamidine, or a salt thereof is administered once daily and at least one compound selected from the group consisting of etidronic acid, clodronic acid, pamidronic acid, tiludronic acid, risedronic acid, minodronic acid, ibandronic acid, zoledronic acid, and salts thereof is administered once weekly or biweekly or twice monthly. Further, when both drugs are single drugs, they may be administered at ordinary intervals for the respective drugs in combination. Even when it is selected to administer the respective drugs at different intervals, convenience is expected to be improved by using the form of a kit such as blister package and the like.

N-hydroxy-4-{5-[4-(5-isopropyl-2-methyl-1,3-thiazol-4-yl)phenoxy]pentoxy}-benzamidine, or a salt thereof; and at least one compound selected from the group consisting of etidronic acid, clodronic acid, pamidronic acid, tiludronic acid, risedronic acid, minodronic acid, ibandronic acid, zoledronic acid, and salts thereof according to the present invention may be formulated themselves alone or together with appropriate excipients described below by known methods. Specific examples of the dosage form include oral preparations such as soft capsules, hard capsules, tablets, syrups and the like; parenteral injections; and drugs for external use.

Examples of the excipients include vegetable oils (for example, corn oil, cotton seed oil, coconut oil, almond oil, and peanut oil), oily esters such as medium chain fatty acid glycerides and the like, mineral oils, Vaseline, animal fats and oils, cellulose derivatives (for example, crystalline cellulose, hydroxypropyl cellulose, hydroxypropylmethyl cellulose, and methyl cellulose), polyvinyl pyrrolidone, dextrin, lactose, mannitol, sorbitol, starch and the like. In addition, additives such as antioxidants, wetting agents, viscosity stabilizers, colorants and the like may be added, as required.

Further, the present inventions is not restricted to a preventive or therapeutic agent or a preventive or therapeutic method for osteoporosis and may be applied to a preventive or therapeutic agent or a preventive or therapeutic method for diseases caused by abnormal bone metabolism, especially diseases caused by abnormal bone resorption among abnormal bone metabolism. The diseases caused by abnormal bone resorption include rheumatoid arthritis, bone Paget's disease, hypercalcemia, periodontal bone loss, renal osteodystrophy, osteolytic tumor, bone metastatic tumor and the like. The explanation about the preventive or therapeutic agent or the preventive or therapeutic method for osteoporosis can also be applied to the preventive or therapeutic agent or the preventive or therapeutic method for these diseases.

### Examples

### Example 1: Study of the effects of N-hydroxy-4-{5-[4-(5-isopropyl-2-methyl-1,3-thiazol-4-yl)phenoxy]pentoxy}-benzamidine and risedronic acid on the formation of osteoclast-like cells

Multinuclear osteoclast-like cells were formed from mouse bone marrow-derived cells by collecting bone marrows of the femur and the tibia from a mouse, removing erythrocytes according to the ordinary method, plating the residual components in a 96-well plate at the density of 4x10⁵ cells/well, culturing the cells overnight, then adding a medium containing 1α,25-dihydroxy vitamin D₃ (referred to as 1α,25(OH)₂D₃ hereinbelow) in order to be at 10⁻⁹ M, and culturing the cells in the presence of 1α,25(OH)₂D₃ for 7 days. An αMEM medium containing 10% fetal calf serum (referred to as 10% FCS-αMEM hereinbelow) was used as a medium. When 1α,25(OH)₂D₃ was added to the mouse bone marrow-derived cells, each of a solvent alone (Comparative Example 1-1), N-hydroxy-4-{5-[4-(5-isopropyl-2-methyl-1,3-thiazol-4-yl)phenoxy]pentoxy}-benzamidine (described as Compound A in Figure) alone (Comparative Example 1-2), risedronic acid alone (Comparative Example 1-3), and N-hydroxy-4-{5-[4-(5-isopropyl-2-methyl-1,3-thiazol-4-yl)phenoxy]pentoxy}-benzamidine (described as Compound A in Figure) and risedronic acid simultaneously (Example 1) were added to the mouse bone marrow-derived cell culture system, and the erects on formation of osteoclast-like cells were evaluated. The following groups were set for the experiment.

### Comparative Example 1-1: Control Group (control)

### Comparative Example 1-2: Group in which N-hydroxy-4-{5-[4-(5-isopropyl-2-methyl-1,3-thiazol-4-yl)phenoxy]phenoxy}-benzamidine (Compound A) at 10⁻⁷ M alone was added

### Comparative Example 1-3: Group in which risedronic acid at 3 x 10⁻⁷ M alone was added

### Example 1: Group in which N-hydroxy-4-{5-[4-(5-isopropyl-2-methyl-1,3-thiazol-4-yl)phenoxy]pentoxy}- benzamidine (Compound A) at 10⁻⁷ M and risedronic acid at 3 x 10⁻⁷ M were added simultaneously

For addition of N-hydroxy-4-{5-[4-(5-isopropyl-2-methyl-1,3-thiazol-4-yl)phenoxy]pentoxy}-benzamidine (Compound A), in both the Comparative Examples and Example, N-hydroxy-4-{5-[4-(5-isopropyl-2-methyl-1,3-thiazol-4-yl)phenoxy]pentoxy}-benzamidine (Compound A) was dissolved in dimethylsulfoxide (DMSO) at a concentration of 10⁻⁴ M, and the resultant solution was added to the medium to make 10⁻⁷ M at a 1000-fold dilution. For addition of risedronic acid, in both the Comparative Examples and Example, sodium risedronate hydrate was dissolved in phosphate buffered saline (PBS) at a concentration of 3 x 10⁻⁴ M, and the resultant solution was added to the medium to make 3 x 10⁻⁷ M at a 1000-fold dilution. On the third day after addition of the test compound(s) of the respective conditions, the medium was replaced with fresh 10% FCS-αMEM containing the test compound(s) of the respective conditions in both the Comparative Examples and Example.

In both the Comparative Examples and Example, on the 7th day after the start of culture, the medium was removed, the cells were fixed with phosphate buffered formalin and stained by tartrate-resistant acid phosphatase (abbreviated as TRAP hereinbelow), an osteoclast marker, using Sigma Acid Phosphatase, Leukocyte (TRAP) kit (Sigma, No. 386A), and the number of TRAP-positive multinuclear cells having 6 or more cell nuclei was counted as osteoclast-like cells under an optical microscope.

The results are shown in Figure 1. In the graph, the percentages of the number of osteoclast-like cells formed in each of the Comparative Examples and Example, taking the number of osteoclast-like cells formed in Comparative Example 1-1 as 100%, are shown using the values shown as the average ± standard deviation of 3 wells for each group. In Comparative Example 1-2 (in which N-hydroxy-4-{5-[4-(5-isopropyl-2-methyl-1,3-thiazol-4-yl)phenoxy]pentoxy}-benzamidine (Compound A) alone was added) and Comparative Example 1-3 (in which risedronic acid alone was added), the numbers of osteoclast-like cells showed a tendency to decrease (Comparative Example 1-2: 81.3% and Comparative Example 1-3: 88.1%) as compared with that in Comparative Example 1-1 (the control group in which the solvent was added), with no significant difference.

As shown in Figure 1, the number of osteoclast-like cells greatly decreased in Example 1 (in which N-hydroxy-4-{5-[4-(5-isopropyl-2-methyl-1,3-thiazol-4-yl)phenoxy]pentoxy}-benzamidine (Compound A) and risedronic acid were added simultaneously) (52.3%) as compared with those in Comparative Example 1-2 and Comparative Example 1-3, and was significantly lower than that in Comparative Example 1-1. In other words, when the above two agents were used in combination, a significantly stronger effect was observed than the effect expected from the sum of the effects of the respective single agents.

In the Figure, * shows p<0.05 as compared with the control group by the Dunnett's t-test; *** shows p<0.001 as compared with the control group by the Dunnett's t-test; # shows p<0.05 as compared with Comparative Example 2-2 by the Student's t-test; and ## shows p<0.01 as compared with Comparative Example 2-3 by the Student's t-test.

The osteoclast-like cell formation inhibition rates of the respective groups with respect to Comparative Example 1-1 are shown in Table 1.

**[Table 1]**

| Effects of N-hydroxy-4-{5-[4-(5-isopropyl-2-methyl-1,3-thiazol-4-yl)phenoxy] pentoxy}-benzamidine and risedronic acid on the formation of osteoclast-like cells | |
|---|---|
| | Osteoclast-like cell formation inhibition rate (%) |
| Comparative Example 1-1 | 0.0 |
| Comparative Example 1-2 | 18.7 |
| Comparative Example 1-3 | 11.9 |
| Example 1 | 47.7 |

### Example 2: Study of the effects of N-hydroxy-4-{5-[4-(5-isopropyl-2-methyl-1,3-thiazol-4-yl)phenoxy]pentoxy}-benzamidine and risedronic acid on the formation of osteoclasts

The experiment was conducted in a similar manner to that described in Example 1, except that the concentration of N-hydroxy-4-{5-[4-(5-isopropyl-2-methyl-1,3-thiazol-4-yl)phenoxy]pentoxy}-benzamidine in the medium was set at 10⁻⁶ M, and the following groups were set.

### Comparative Example 2-1: Control Group (control)

### Comparative Example 2-2: Group in which N-hydroxy-4-{5-[4-(5-isopropyl-2-methyl-1,3-thiazol-4-yl)phenoxy]pentoxy}-benzamidme (Compound A) at 10⁻⁶ M alone was added

### Comparative Example 2-3: Group in which risedronic acid at 3×10⁻⁷ M alone was added

### Example 2: Group in which N-hydroxy-4-{5-[4-(5-isopropyl-2-methyl-1,3-thiazol-4-yl)phenoxy]pentoxy}-benzamidine (Compound A) at 10⁻⁶ M and risedronic acid at 3 x 10⁻⁷ M were added simultaneously

The results are shown in Figure 2. In the graph, the percentages of the number of osteoclast-like cells formed in each of the Comparative Examples and Example, taking the number of osteoclast-like cells formed in Comparative Example 2-1 as 100%, are shown using the values shown as the average ± standard deviation of 3 wells for each group. In Comparative Example 2-2 (in which (N-hydroxy-4-{5-[4-(5-isopropyl-2-methyl-1,3-thiazol-4-yl)phenoxy]pentoxy}-benzamidine (Compound A) alone was added), the number of osteoclast-like cells significantly decreased (Comparative Example 2-2; 50%) as compared with that in Comparative Example 2-1 (the control group in which the solvent was added). In Comparative Example 2-3 (in which risedronic acid alone was added), however, the number of osteoclast-like cells showed a tendency to decrease (Comparative Example 2-3; 88.1%) as compared with that in Comparative Example 2-1 (the control group in which the solvent was added), with no significant difference. In Example 2 (in which N-hydroxy-4-{5-[4-(5-isopropyl-2-methyl-1,3-thiazol-4-yl)phenoxy]pentoxy}-benzamidine (Compound A) and risedronic acid were added simultaneously), the number of osteoclast-like cells remarkably decreased (10.8%) as compared with those in Comparative Example 2-2 and Comparative Example 2-3, with a significantly lower value as compared with that in Comparative Example 2-1 or Comparative Example 2-3. In other words, when the above two agents were used in combination, a significantly stronger effect was observed than the effect expected from the sum of the effects of the respective single agents. In the Figure, * shows p<0.05 as compared with the control group by the Dunnett's t-test; *** shows p<0.001 as compared with the control group by the Dunnett's t-test; # shows p<0.05 as compared with Comparative Example 2-2 by the Student's t-test; and ## shows p<0.01 as compared with Comparative Example 2-3 by the Student's t-test.

The osteoclast-like cell formation inhibition rates of the respective groups with respect to Comparative Example 2-1 are shown in Table 2.

**[Table 2]**

| Effects of N-hydroxy-4-{5-[4-(5-isopropyl-2-methyl-1,3-thiazol-4-yl)phenoxy] pentoxy}-benzamidine and risedronic acid on the formation of osteoclast-like cells | |
|---|---|
| | Osteoclast-like cell formation inhibition rate (%) |
| Comparative Example 2-1 | 0.0 |
| Comparative Example 2-2 | 50.0 |
| Comparative Example 2-3 | 11.9 |
| Example 2 | 89.2 |

### Example 3: Study of the effects of N-hydroxy-4-{5-[4-(5-isopropyl-2-methyl-1,3-thiazol-4-yl)phenoxy]pentoxy}-benzamidme and clodronic acid on the formation of osteoclast-like cells

The experiment was conducted in a similar manner to that described in Example 1, except that clodronic acid at a concentration of 10⁻⁵ M was allowed to act on the cells in place ofrisedronic acid at a concentration of 3×10⁻⁷ M in the Comparative Examples and Example, and the following groups were set.

### Comparative Example 3-1: Control Group (control)

### Comparative Example 3-2: Group in which N-hydroxy-4-{5-[4-(5-isopropyl-2-methyl-1,3-thiazol-4-yl)phenoxy]pentoxy}-benzamidine (Compound A) at 10⁻⁷ M alone was added

### Comparative Example 3-3: Group in which clodronic acid at 10⁻⁵ M alone was added

### Example 3: Group in which N-hydroxy-4-{5-[4-(5-isopropyl-2-methyl-1,3-thiazol-4-yl)phenoxy]pentoxy}-benzamidine (Compound A) at 10⁻⁷ M and clodronic acid at 10⁻⁵ M were added simultaneously

The results are shown in Figure 3. In the graph, the percentages of the number of osteoclast-like cells formed in each of the Comparative Examples and Example, taking the number of osteoclast-like cells formed in Comparative Example 3-1 as 100%, are shown using the values shown as the average ± standard deviation of 4 to 5 wells for each group. In Comparative Example 3-2 (in which N-hydroxy-4-{5-[4-(5-isopropyl-2-methyl-1,3-thiazol-4-yl)phenoxy]pentoxy}-benzamidine (Compound A) alone was added), the number of osteoclast-like cells showed a tendency to decrease (Comparative Example 3-2; 95.2%) as compared with that in Comparative Example 3-1 (control group in which the solvent was added), with no significant difference. In Comparative Example 3-3 (in which clodronic acid alone was added), however, the number of osteoclast-like cells showed a tendency to decrease (Comparative Example 3-3; 93.9%) as compared with that in Comparative Example 3-1 (control group in which the solvent was added), with no significant difference. In Example 3 (in which N-hydroxy-4-{5-[4-(5-isopropyl-2-methyl-1,3-thiazol-4-yl)phenoxy]pentoxy}-benzamidine (Compound A) and risedronic acid were added simultaneously), the number of osteoclast-like cells decreased markedly (57.3%) as compared with those in Comparative Example 3-2 and Comparative Example 3-3, and was significantly lower than those in Comparative Example 3-1, Comparative Example 3-2 and Comparative Example 3-3. In other words, when the above two agents were used in combination, a significantly stronger effect was observed than the effect expected from the sum of the effects of the respective single agents.

In the Figure, * shows p<0.05 as compared with the control group by the Dunnett's t-test; # shows p<0.05 as compared with Comparative Example 3-2 by the Student's t-test; and ## shows p<0.01 as compared with Comparative Example 3-3 by the Student's t-test.

The osteoclast-like cell formation inhibition rates of the respective groups with respect to Comparative Example 3-1 are shown in Table 3.

**[Table 3]**

| Effects of N-hydroxy-4-{5-[4-(5-isopropyl-2-methyl-1,3-thiazol-4-yl)phenoxy] pentoxy}-benzamidine and clodronic acid on the formation of osteoclast-like cells | |
|---|---|
| | Osteoclast-like cell formation inhibition rate (%) |
| Comparative Example 3-1 | 0.0 |
| Comparative Example 3-2 | 4.8 |
| Comparative Example 3-3 | 6.1 |
| Example 3 | 42.7 |

### Example 4: Study of the effects of N-hydroxy-4-{5-[4-(5-isopropyl-2-methyl-1,3-thiazol-4-yl)phenoxy]pentoxy}-benzamidine and risedronic acid on the bone resorption activity of osteoclast-like cells

Multinuclear osteoclast-like cells were formed from mouse bone marrow-derived cells by collecting bone marrows of the femur and the tibia from a mouse, removing erythrocytes according to the ordinary method, plating the residual components in a 96-well plate containing an ivory section at the density of 4 x 10⁵ cells/well, culturing the cells overnight, then adding a medium containing 1α,25-dihydroxy vitamin D₃ (referred to as 1α,25(OH)₂D₃ hereinbelow) in order to be at 10⁻⁹ M, and culturing the cells in the presence of 1α,25(OH)₂D₃ for 9 days. An αMEM medium containing 10% fetal calf serum (referred to as 10% FCS-αMEM hereinbelow) was used as a medium. When 1α,25(OH)₂D₃ was added to the mouse bone marrow-derived cells, each of a solvent alone (Comparative Example 4-1), N-hydroxy-4-{5-[4-(5-isopropyl-2-methyl-1,3-thiazol-4-yl)phenoxy]pentoxy}-benzamidine (described as Compound A in Table 4) alone (Comparative Example 4-2), risedronic acid alone (Comparative Example 4-3), and N-hydroxy-4-{5-[4-(5-isopropyl-2-methyl-1,3-thiazol-4-yl)phenoxy]pentoxy}-benzamidine (described as Compound A in Table 4) and risedronic acid (Example 4) were simultaneously added to the mouse bone marrow-derived cell culture system, and the effects on formation of osteoclast-like cells were evaluated. The following groups were set for the experiment.

### Comparative Example 4-1: Control Group (control)

### Comparative Example 4-2: Group in which N-hydroxy-4- {5-[4-(5-isopropyl-2-methyl-1,3-thiazol-4-yl)phenoxy]pentoxy}-benzamidine (Compound A) at 10⁻⁷ M alone was added

### Comparative Example 4-3: Group in which risedronic acid at 10⁻⁸ M alone was added

### Example 4: Group in which N-hydroxy-4-{5-[4-(5-isopropyl-2-methyl-1,3-thiazol-4-yl)phenoxy]pentoxy}- benzamidine (Compound A) at 10⁻⁷ M and risedronic acid at 10⁻⁸ M were added simultaneously

For addition of N-hydroxy-4-{5-[4-(5-isopropyl-2-methyl-1,3-thiazol-4-yl)phenoxy]pentoxy}-benzamidine (Compound A), in both the Comparative Examples and Example, N-hydroxy-4-{5-[4-(5-isopropyl-2-methyl-1,3-thiazol-4-yl)phenoxy]pentoxy}-benzamidine (Compound A) was dissolved in dimethylsulfoxide (DMSO) at a concentration of 10⁻⁴ M, and the resultant solution was added to the medium to make 10⁻⁷ M at a 1000-fold dilution. For addition of risedronic acid, in both the Comparative Examples and Example, sodium risedronate hydrate was dissolved in phosphate buffered saline (PBS) at a concentration of 10⁻⁵ M, and the resultant solution was added to the medium to make 10⁻⁸ M at a 1000-fold dilution. On every 3 to 4 day after addition of the test compound(s) of the respective conditions, the medium was replaced with fresh 10% FCS-αMEM containing the test compound(s) of the respective conditions in both the Comparative Examples and Example.

Both in Comparative Examples and Example, the culture medium was collected 9 days after the start of culture, and the quantity of type I collagen cross-linked C-telopeptide in the medium was measured. Crosslaps for Culture ELISA (Nordic Bioscience Diagnostics, No. 6CRL4000) was used for the measurement. Type I collagen cross-linked C-telopeptide is a peptide fragment produced by degradation of ivory by the bone resorption activity of osteoclast-like cells.

The results are shown in Table 4. In Table 4, the inhibitory rate is shown by the percentage of the quantity of type I collagen cross-linked C-telopeptide produced in each group with respect to that in Comparative Example 4-1 using the average values of 3 to 4 wells for each group. In Comparative Example 4-2 (in which N-hydroxy-4-{5-[4-(5-isopropyl-2-methyl-1,3-thiazol-4-yl)phenoxy]pentoxy}-benzamidine (Compound A) alone was added) and Comparative Example 4-3 (in which risedronic acid alone was added), the number of osteoclast-like cells showed a tendency to decrease (Comparative Example 4-2; 13.9%, Comparative Example 4-3; 18.32%) as compared with that in Comparative Example 4-1 (control group in which the solvent was added), with no significant difference.

**[Table 4]**

| Effects of N-hydroxy-4-{5-[4-(5-isopropyl-2-methyl-1,3-thiazol-4-yl)phenoxy] pentoxy}-benzamidine and risedronic acid on the bone resorption activity of osteoclast-like cells | |
|---|---|
| | Bone resorption inhibitory rate (%) |
| Comparative Example 4-1 | 0.0 |
| Comparative Example 4-2 | 13.9 |
| Comparative Example 4-3 | 18.3 |
| Example 4 | 38.7 (*) |

As shown in Table 4, the number of osteoclasts in Example 4 (in which N-hydroxy-4-{5-[4-(5-isopropyl-2-methyl-1,3-thiazol-4-yl)phenoxy]pentoxy}-benzamidine (Compound A) and risedronic acid were added simultaneously) decreased remarkably (38.7%) as compared with those in Comparative Example 4-2 and Comparative Example 4-3, and was significantly lower than that in Comparative Example 4-1. In other words, when the above two agents were used in combination, a significantly stronger effect was observed than the effect expected from the sum of the effects of the respective single agents.

In the Figure, * shows p<0.05 as compared with Comparative Example 4-1 by the Dunnett's t-test.

### Industrial Applicability

The present invention can be used as a preventive or therapeutic drug or a preventive or therapeutic method for diseases caused by abnormal bone metabolism, especially osteoporosis by administration of N-hydroxy-4-{5-[4-(5-isopropyl-2-methyl-1,3-thiazol-4-yl)phenoxy]pentoxy}-benzamidine, or a salt thereof and at least one compound selected from the group consisting of etidronic acid, clodronic acid, pamidronic acid, tiludronic acid, risedronic acid, minodronic acid, ibandronic acid, zoledronic acid, and salts thereof in combination.

## Claims

1. A preventive or therapeutic agent for diseases caused by abnormal bone metabolism, comprising as active ingredients the following (a) and (b):
(a) N-hydroxy-4-{5-[4-(5-isopropyl-2-methyl-1,3-thiazol-4-yl)phenoxy]pentoxy}-benzamidine, or a salt thereof; and
(b) at least one compound selected from the group consisting of etidronic acid, clodronic acid, pamidronic acid, tiludronic acid, risedronic acid, minodronic acid, ibandronic acid, zoledronic acid, and salts thereof

2. The preventive or therapeutic agent for diseases caused by abnormal bone metabolism according to Claim 1, wherein the diseases caused by abnormal bone metabolism are diseases caused by abnormal bone resorption.

3. The preventive or therapeutic agent for diseases caused by abnormal bone metabolism according to Claim 1 or 2, wherein the disease caused by abnormal bone metabolism is osteoporosis.

4. The preventive or therapeutic agent for diseases caused by abnormal bone metabolism according to any one of Claims 1 to 3, wherein (b) is clodronic acid or risedronic acid or a salt thereof

5. The preventive or therapeutic agent for diseases caused by abnormal bone metabolism according to any one of Claims 1 to 3, wherein (b) is risedronic acid or a salt thereof.

6. The preventive or therapeutic agent for diseases caused by abnormal bone metabolism according to any one of Claims 1 to 5, wherein (a) and (b) form a combination drug.

7. The preventive or therapeutic agent for diseases caused by abnormal bone metabolism according to any one of Claims 1 to 5, wherein (a) and (b) each are independent single drugs.

8. The preventive or therapeutic agent for diseases caused by abnormal bone metabolism according to anyone of Claims 1 to 5, wherein (a) and (b) are contained in a kit.
